# EUROPEAN PATENT SPECIFICATION

(11) **EP 0 309 560 B1**
(45) Date of publication and mention of the grant of the patent: **21.12.1994**
(21) Application number: 88903972.3
(22) Date of filing: 06.04.1988
(51) Int. Cl.: C07H 19/06, C07H 19/073, C07D 239/46, C07D 405/04, C07D 409/04, A61K 31/70

(54) **NUCLEOSIDES AND NUCLEOSIDE ANALOGUES, PHARMACEUTICAL COMPOSITION AND PROCESSES FOR THE PREPARATION OF THE COMPOUNDS**
NUKLEOSIDE UND NUKLEOSID-ANALOGE, PHARMAZEUTISCHE ZUSAMMENSETZUNG UND VERFAHREN FÜR DIE HERSTELLUNG DER BESTANDTEILE
NUCLEOSIDES ET ANALOGUES DE NUCLEOSIDES, COMPOSITIONS ET PROCEDES PHARMACEUTIQUES PERMETTANT LA PREPARATION DES COMPOSES

(30) Priority: 16.04.1987 SE 8701605
(43) Date of publication of application: 05.04.1989
(60) Divisional application: 92112530.8
(73) Proprietor: Medivir Aktiebolag, S-141 44 Huddinge (SE)
(72) Inventor: DATEMA, Roelf, S-151 44 Södertälje (SE); KOVACS, Zsuzanna, Maria, Ilona, I-17027 Pietra Ligure (IT); JOHANSSON, Karl, Nils, Gunnar, S-150 23 Enhörna (SE); LINDBORG, Björn, Gunnar, S-125 42 Älvsjö (SE); STENING, Göran, Bertil, S-151 50 Södertälje (SE); ÖBERG, Bo, Fredrik, S-752 52 Uppsala (SE)
(74) Representative: Dost, Wolfgang, Dr.rer.nat., Dipl.-Chem.
(86) International application number: SE8800169
(87) International publication number: WO8808001

(56) References cited:
- EP-A- 0 196 185
- EP-A- 0 199 451
- EP-A- 0 206 497
- EP-A- 0 217 580
- DD-A- 75 084
- DE-A- 2 918 260
- DE-A- 2 930 904
- DE-A- 3 002 197
- DE-A- 3 045 375
- DE-A- 3 229 168
- FR-A- 2 040 177
- JP-A-56 322 729
- US-A- 3 116 282
- US-A- 3 817 982
- US-A- 4 247 544
- US-A- 4 267 171
- Nucleosides and Nucleotides 1 (1982) 263-273
- J Med Chem 33 (1990) 2494-2501
- Journal of Medicinal Chemistry, Vol 17, No 3,1974, T Kulikowski et al, "5 -Alkylpyrimidine Nucleosides. Preparation and Properties of 5-Ethyl-2' - deoxycytidine and Related Nucleosides", see p. 269-273
- Chemical Abstracts, Vol 102 (1985), abstract No 7013q, Chem. Pharm. Bull. 1984,32(4), 1441-50 (Eng)
- Chemical Abstracts, Vol. 92 (1980), abstract No 208903d, Vopr. Virusol. 17979, (6). 603-6 (russ)
- Chemical society, London. Journal. Perkin trans. I, 1978, P.J. Barr et al, "The Synthesis of Nucleosides derived from 5-Ethynyluracil and 5-Ethynyl-cytosine" see p. 1263-1267
- J. Carbohydrates. Nucleosides. Nucleotide. Vol. 5, No 3, 1978, E DeClerq et al, "Nucleoside analoge with selective antiviral activity", see p 187-224

## Description

The present invention relates to a chemical compound and to its use and that of physiologically acceptable salts of this compound for the therapeutic and prophylactic control and treatment of the Acquired Immuno Deficiency syndrome (AIDS), infections by Human Immunodeficiency Virus, hepatitis B virus infections and retrovirus infections, and to a method for such control and treatment in animal and man.

### Background of the invention

In the late seventies a new disease was reported, which subsequently was referred to as Acquired Immuno Deficiency Syndrome (AIDS). It is now generally accepted that a retrovirus referred to as HIV (Human Immunodeficiency Virus), formerly known as Human T-cell Lymphotropic Virus (HTLV-III) or Lymphadenopathy Associated Virus (LAV) plays an essential role in the etiology of AIDS.

AIDS is characterized by a profound immunodeficiency due to low numbers of a subset of lymphocyte-T-helper cells, which are one target for HIV infection. The profound imunodeficiency in AIDS patients makes these patients highly susceptible to a variety of opportunistic infections of bacterial, fungal, protozoal or viral etiology. The etiological agents among viral opportunistic infections are often found in the herpes virus group, i.e., Herpes simplex virus (HSV), Varicella Zoster virus (VZV), Epstein-Barr virus (EBV) and, especially, cytomegalovirus (CMV). Other retroviruses affecting humans are HTLV-I and II and examples of retroviruses affecting animals are feline leukemia virus and equine infectious anaemia virus.

Hepatitis B virus infections cause severe disease such as acute hepatitis, chronic hepatitis, fulminant hepatitis in a considerable number of persons. It is estimated that there are 200 million patients with chronic hepatitis B infection in the world. A considerable number of the chronic cases progress to liver cirrosis and liver tumours. In some cases the hepatitis infections also take a rapid and severe course as in fulminant B hepatitis with about 90 % mortality. At present there is no known effective treatment against hepatitis B infections.

### General outline of the invention

A great number of nucleoside analogues exhibit several antimetabolic activities. They do so by substituting for or competing with the naturally occuring nucleosides. Recently some nucleoside analogues have been described, which inhibit in cell culture the multiplication of human immunodeficiency virus (HIV, also called HTLV-III, LAV), the causative agent of AIDS and AIDS-related complex (ARC). Such compounds are for example azidothymidine, dideoxycytidine and dideoxyadenosine. These and other described HIV-antimetabolic nucleoside analogues have the same geometric relationship between the nucleoside base and the glycosidic part as the naturally occuring nucleosides, i.e. they are β-anomers.

We have now, surprisingly, found that some nucleosides and nucleoside analogues with the opposite geometric configuration, α-anomers, are potent inhibitors of HIV multiplication but not of cell-division. Anti-HIV activities are displayed by such geometric isomers which have been modified either in the nucleoside base part, the glycoside part or in both parts. The structures of these compounds are disclosed in this invention.

### Prior Art

The following compounds having a structural formula similar to the formula I below are known:
1. Compounds of the formula 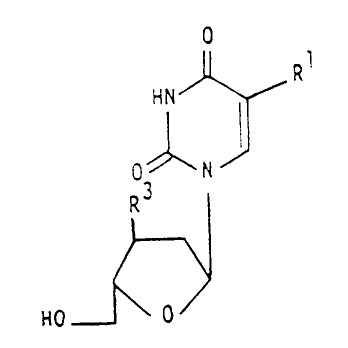 wherein R³ is OH and R¹ is as follows:
   - R¹ is H and CH₃:: T. Nishimura, B. Shinizu, I. Iwai Chem. Pharm. Bull. (Tokyo) 12 (1964) , 1471;
   - R¹ is C₂H₅:: M. Swierkowski, D. Shugar J. Med. Chem. 12 (1969), 533;
   - R¹ is n-C₃H₇:: A. Szaboles, J. Sági, L. Ötvös J. Carbohydrates, Nucleosides, Nucleotides 2 (1975), 197 - 211;
   - R¹ is i-C₃H₇:: M. Draminski, A. Zgit-Wroblewska Polish J. Chemistry 54 (1980), 1085;
   - R¹ is C≡CH:: P.J. Barr , A.S. Jones , P. Serafinowski, R. Walker; J. Chem. Soc. Perkin I (1978), 1263 - 1267;
   and wherein R³ is N₃ and R¹ is CH₃: M. Imezawa, F. Eckstein, J. Org. Chem. 43 (1978), 3044-3048.
2. The compound of the formula 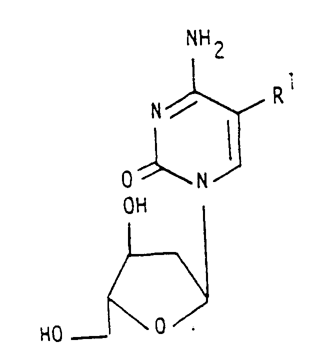 wherein R¹ is C≡CH is described by P.J. Barr, A.S. Jones, P. Serafinowski, R. Walker, J. Chem. Soc. Perkin I (1978), 1263 - 1267;
R¹ is H is described by J.J. Fox, N.C. Yung, I. Wempen and M. Hoffer, J. Am. Chem. Soc., Vol. 83 (1961), 4066-4070.
Both groups 1. and 2. concern only compounds having the 3'-group and the 4'-hydroxymethyl group in a trans-configuration.
Additional compounds of the formula under 2. as well as under 1., wherein R³ is OH, are known from the following references
   J. Med. Chem. 17 (1974) 269-273,
   DE-A-30 02 197, DE-A-32 29 169,
   US-A-3,116,282,
   Chem. Pharm. Bull 32 (1984) 1441-50 (Chem. Abst. 102, 7013q),
   Vopr. Virusol. 1979 (6) 603-6 (Chem. Abst. 92, 208903d),
   DE-A-29 30 904, DE-A-29 18 260, DE-A-30 45 375,
   US 4,247,544, US 4,267,171,
   J. Carbohydr. Nucleos. Nucleot. 5 (1978) 187-224, FR-A-2 040 177.
3. Compounds of the formula 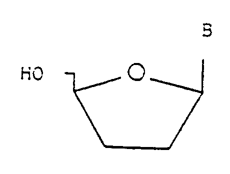 wherein B is a purine or cytosine base are known from EP 0 206 497 and US 3 817 982.
4. Compounds of the formula 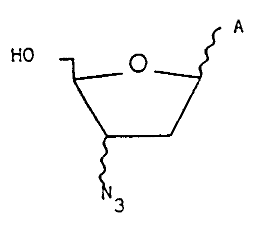 wherein A is a purine or pyrimidine base are known from EP 0 217 580, EP 0 196 185 and EP 0 199 451.
5. Compounds of the formulas 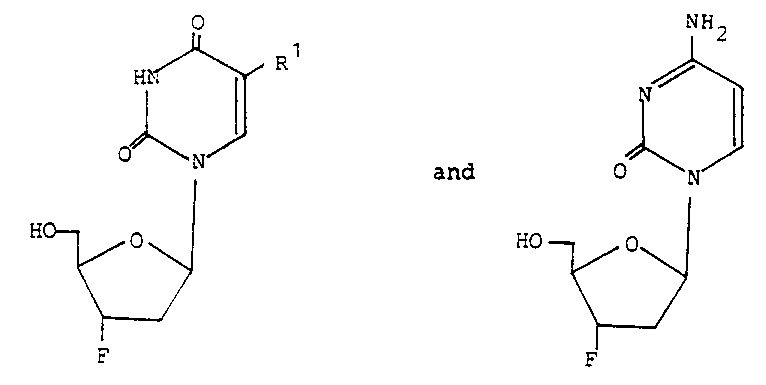 wherein R¹ is H, CH₃, C₂H₅, are known from at least one of EP 0 277 151, US 3,775,397, EP 0 254 268, DD 75084 or GB 1,189,973.
6. Compounds of the formula 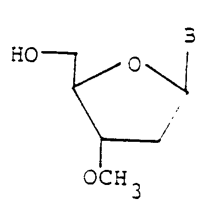 wherein B is thymine or cytosine are known from Yamauchi et al, J. Chem Soc. Perkin Trans. 1, (1980) 2787-2792.
7. Other compounds comprising as the glycosidically linked base the cytosine residue are known from at least one of Darzynkiewicz et al., Biochem. Biophys. Res. Comm. 46, 1734 (1972) and Remin and Shugar, JACS 95, 8146 (1973). 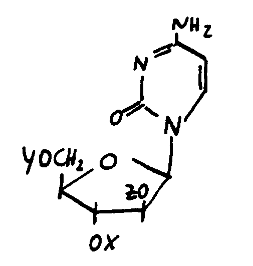 wherein each of X, Y and Z is either H or CH₃.
8. Finally, the compound 2', 3'-dideoxy-3'-(R)-hydroxymethyl uridine having the structural formula. 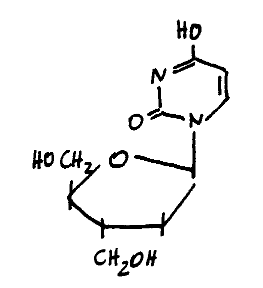 is known from Japanese Laying Open Print No. 57-146798 and from Nucleosides & Nucleotides 1,263-273 (1982).

### Disclosure of the invention

It has been found according to the present invention that the compound of the formula 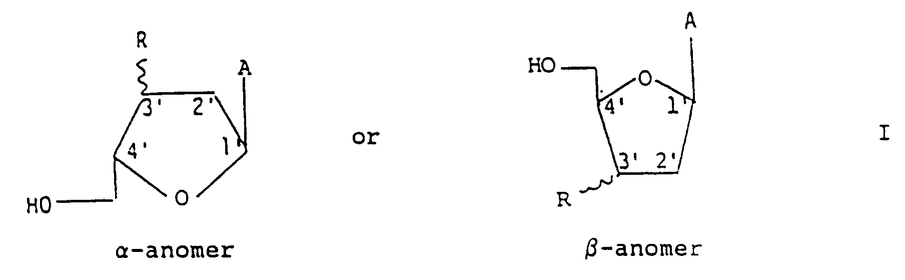
wherein the radicals A and R are defined as follows: 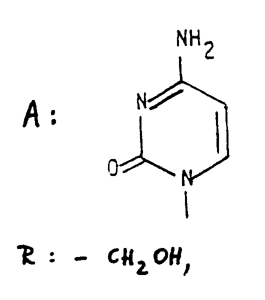
wherein R may have either cis- or trans- configugration relative to the -CH₂OH group located at the 4' position, and therapeutically acceptable salts thereof, inhibits the multiplication of human immunodeficiency virus (HIV). The compound of the formula I is useful as therapeutic and/or prophylactic agents in the control and treatment of HIV virus infections in mammals and man.

In a more general aspect, the compound of the formula I is useful as therapeutic and/or prophylactic agents in the control and treatment of infections caused by retroviruses and hepatitis B virus in mammals and man.

All retroviruses, including HIV, require the enzyme reverse transcriptase in their natural cycle of replication.

Hepatitis B virus (HBV) is a DNA virus with a unique circular double-stranded DNA genome which is partly single-stranded. It contains a specific DNA polymerase required for viral replication. This DNA polymerase also acts as a reverse transcriptase during the replication of HBV DNA via an RNA intermediate.

The compound of the formula I inhibit the activity of reverse transcriptase of retroviruses including HIV as well as the activity of DNA polymerase of hepatitis B virus.

The present invention has several aspects:
1. the novel compound of the formula I,
2. pharmaceutical compositions comprising the compound of the formula I as active ingredient,
3. the compound of the formula I for use in therapy,
4. the compound of the formula I for use in the manufacture of a medicament for therapeutic and/or prophylactic treatment of infections caused by a retrovirus, including HIV, or by hepatitis B virus,
Infections in mammals and man caused by retrovirus including HIV or hepatitis B virus can be therapeutically or prophylactically treated by administering to a host in need of such treatment an efficient amount of the compound of the formula I.

It is a preferred aspect of the invention to combat HIV virus infections in man.

An example of preferred compounds is: 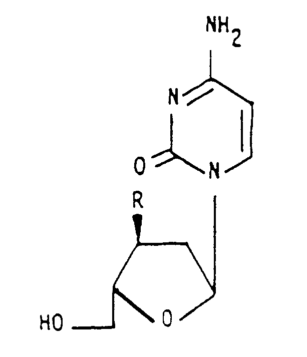
wherein
R is CH₂OH.

In the example of the preferred compound R at position 3' and hydroxymethyl at position 4' have the trans-configuration.

In clinical practice the nucleosides of the formula I will normally be administered orally, by injection or by infusion in the form of a pharmaceutical preparation comprising the active ingredient in the form of the original compound or optionally in the form of a pharmaceutically acceptable salt thereof, in association with a pharmaceutically acceptable carrier which may be a solid, semi-solid or liquid diluent or an ingestible capsule. The compound may also be used without carrier material. As examples of pharmaceutical preparations may be mentioned tablets, dragées, capsules, granulates, suspensions, elixirs, syrups, solutions etc. Usually the active substance will comprise between 0.05 and 20 % for preparations intended for injection and between 10 and 90 % for preparations intended for oral administration.

In the treatment of patients suffering from retrovirus, especially HIV, or hepatitis B virus infections, it will be preferred to administer the compound by any suitable route including the oral, parenteral, rectal, nasal, topical and vaginal route. The parenteral route includes subcutaneous, intramuscular, intravenous and sublingual administration. The topical route includes buccal and sublingual administration. The dosage at which the active ingredients are administered may vary within a wide range and will depend on various factors such as the severity of the infection, the age of patient etc., and may have to be individually adjusted. As a possible range for the amount of the compound of the invention or a physiologically acceptable salt thereof to be administered per day may be mentioned from about 10 mg to about 10 000 mg, preferentially 100 - 500 mg for intravenous administration and preferentially 100 - 3000 mg for oral administration.

Examples of pharmaceutically acceptable salts of the compound of formula I include salts of organic carboxylic acids such as acetic, lactic, gluconic, citric, tartaric, maleic, malic, panthothenic, isethionic, succinic, oxalic, lactobionic and succinic acids; organic sulfonic acids such as methanesulfonic, ethanesulfonic, benzenesulfonic, p-chlorobenzenesulphonic and p-toluenesulfonic acids and inorganic acids such as hydrochloric, hydroiodic, sulfuric, phosphoric and sulfamic acids.

The administered compound may also be used in therapy in conjunction with other medicaments such as 9-[(2-hydroxy-1-(hydroxymethyl)ethoxy)-methyl]guanine, 9-(2-hydroxyethoxymethyl)guanine (acyclovir), 2-amino-9-(2-hydroxyethoxymethyl)purine, interferon, e.g. α-interferon, interleukin II, and phosphonoformate, or in conjunction with immune modulating therapy including bone marrow or lymphocyte transplants or medications such as levamisol or thymosin which would increase lymphocyte numbers and/or function as is appropriate.

### Methods of preparation

The compound of the invention may be prepared by one of the following general methods, constituting a further aspect of the invention.
A. Condensing a glycoside as comprised in formula I, where the hydroxyl groups may be optionally protected, to the N-1 position of a pyrimidine derivative, corresponding to radical A in formula I according to known methods described in the literature, followed by separation of the α-anomer and removal of any protecting group(s). Such methods are described for example in "Basic Principles in Nucleic Acid Chemistry", Vol. 1 (Academic Press, 1974, Ed. P.O.P.Ts'o), in "Nucleoside Analogues", Chemistry, Biology and Medical Applications" (Pharma Press, 1979, Eds. R.T. Walker, E. De Clercq and F. Eckstein) and in Nucleic Acids Research Vol. 12, 1984, pages 6827 - 6837 (A.J. Hubbard, A.S. Jones and R.T. walker). An example of such a method is given for the case of a uracil base analogue: 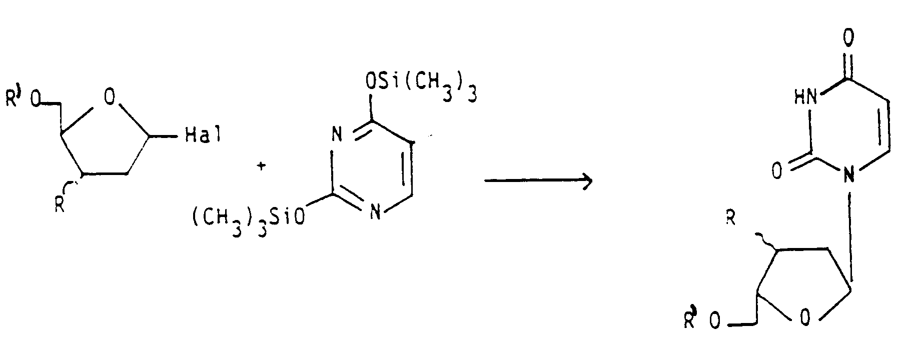 wherein R is CH₂OR', R' is a protecting group, of which a great variety is known, and examples of which are p-toluoyl, acetyl, trityl, benzyl.
B. Anomerization of a β-anomer of the formula 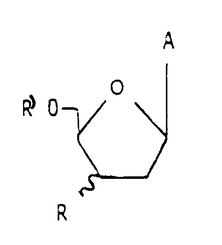 wherein A is as defined above, R is CH₂OR' wherein R' is H or a hydroxy-protecting group, to a mixture of α- and β-anomers, whereafter the α-anomer is separated and any protecting groups removed. The anomerization may be performed by known methods, e.g. with an optionally protected β-nucleoside, for example a silylated nucleoside, with a catalyst, such as for example trimethylsilyl trifluoromethanesulfonate 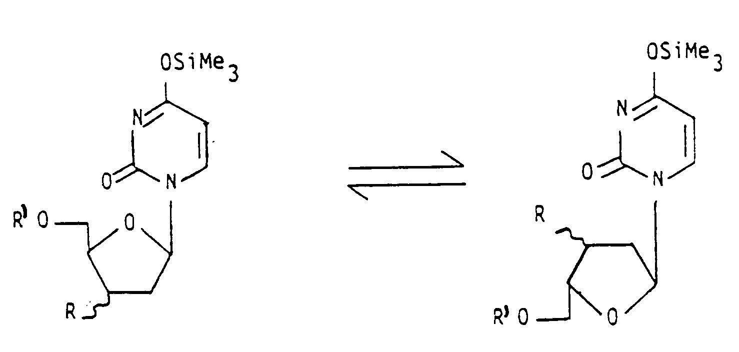 wherein R and R' are as defined above.
C. A transglycosylation reaction whereby the sugar moiety forming a bond, α- or β-, to one nucleoside base, is transferred to the desired pyrimidine base. The reaction is performed with a catalyst such as for example trimethylsilyl trifluoromethanesulfonate, and is followed by separation of the products and deprotection. 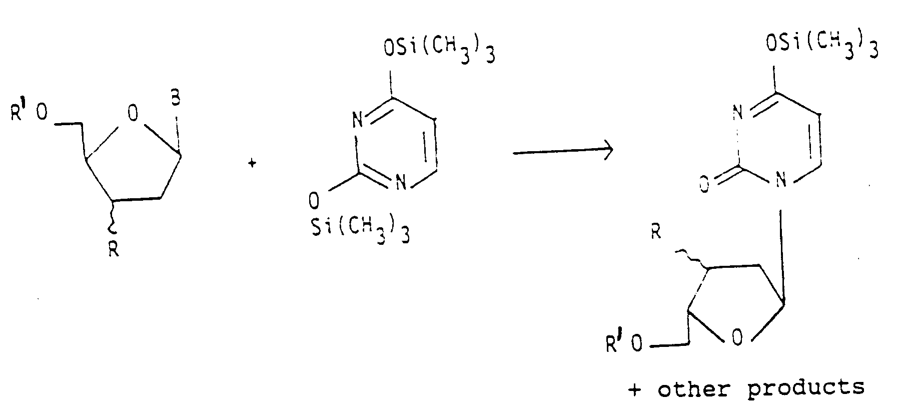 wherein R and R' are as defined above. The radical B is a pyrimidine or purin base, the choice of which is not critical.
D. Introduction of the functional group R^{●}, or a precursor of R^{●}, into the nucleoside α-anomer by substitution of a suitable leaving group, R'', followed by deprotection. 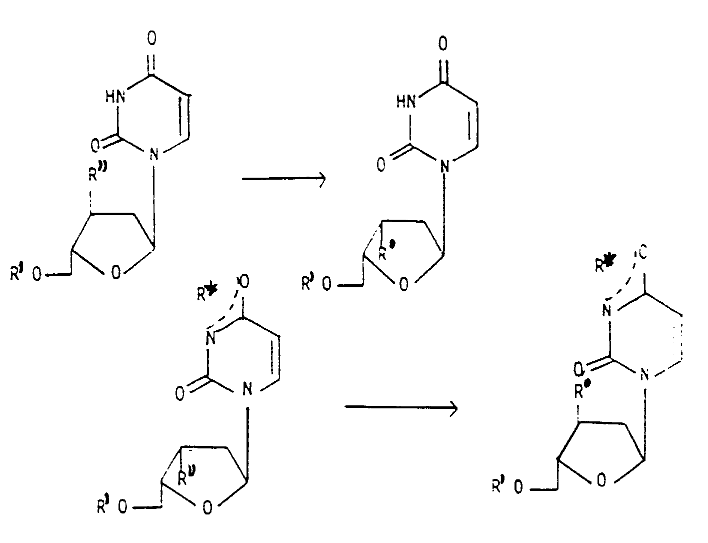 R' is as defined above, R'' is a good leaving group such as for example trifluoromethanesulfonyloxy, R^{●} is a synthon for the CH₂OH group, such as for example 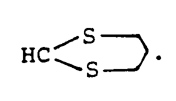 R* is a suitable protecting group.
   An alternative way for introduction of the R^{●} function is by reaction of the 2,3'-anhydro-α-anomer. 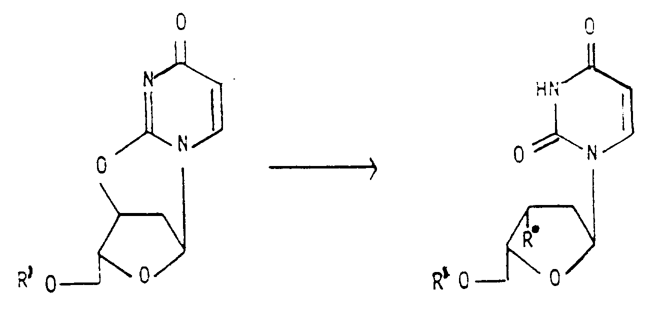 wherein R' and R^{●} are as defined above.
   The principles of methods A-D above are also applicable to the synthesis of cytidine analogues of formula I, although the formula illustrating the reactions only depict uridine analogues.
E. Converting the uracil moiety of the 5-substituted or unsubstituted α-uridine compounds to a cytosine moiety of the corresponding α-cytidine analogues. This is carried out by conventional methods, the principles of which have been described for example by W.L. Sung (J. Chem. Soc. Chem. Commun. 1981, p. 1089 and J. Organic Chemistry 1982, volume 47, pages 3623 - 3628) and by P. Herdewijn et al (J. Medicinal Chemistry 1985, volume 28, pages 550 - 555).

## Claims

1. A compound of the formula 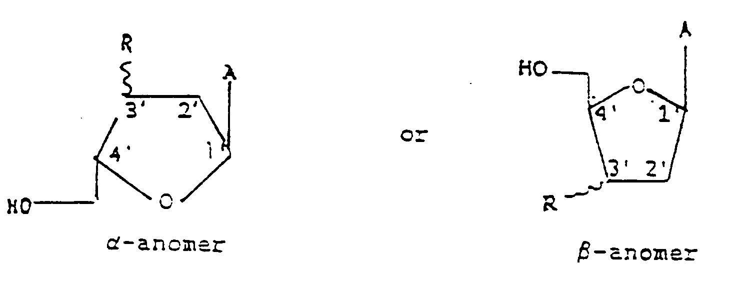 wherein the radicals A and R are as follows:
A: 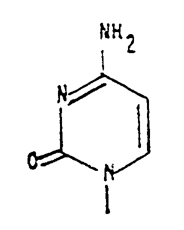
R: = - CH₂OH,
and wherein R may have either cis-configuration or trans-configuration relative to the hydroxy-methyl function at position 4', and therapeutically acceptable salts thereof.

2. A compound according to claim 1 wherein R at position 3' and hydroxy-methyl at position 4' have the trans-configuration.

3. A compound according to claim 1 or 2, and therapeutically acceptable salts thereof, for use in therapy.

4. A pharmaceutical composition comprising as active ingredient a compound of claim 1 or 2 or therapeutically acceptable salts thereof.

5. Use of a compound according to claim 1 or 2, and of therapeutically acceptable salts thereof, in the manufacture of a medicament for therapeutic and/or prophylactic treatment of infections caused by a retrovirus, such as HIV, or by hepatitis B virus (HBV).

6. The use according to claim 5, wherein said therapeutic treatment is against infection in man caused by HIV.

7. The use according to claim 5, wherein said therapeutic treatment is against infection in man caused by HBV.

8. A process for the preparation of a compound according to claim 1 or 2 or of a therapeutically acceptable salt thereof, selected from the group:
A. Condensing a glycoside as comprised in the formula of claim 1 to the N-1 position of the cytosine, whereafter the α-anomer of the compound thus formed is separated and any protecting groups removed;
B. Anomerization of a β-anomer of the formula 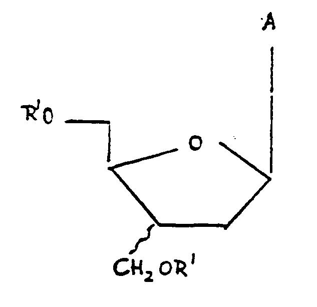 wherein R' is a hydroxy-protecting group, to a mixture of α-and β-anomers, whereafter the α-anomer is separated and any protecting groups removed;
C. Transglycosylation of a nucleoside of the formula 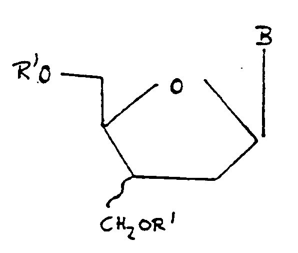 wherein R' is defined as above and wherein B is a pyrimidine or purine base, under formation of a nucleoside containing the cytosine radical as A, whereafter the α-anomer is separated and any protecting groups removed;
D. Substitution of the radical R'' in a compound of the formula 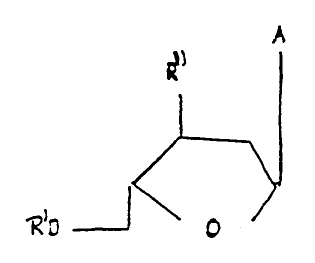 wherein A and R' are as defined above and wherein R'' is a leaving group, with the radical -CH₂OR' as R, whereafter any protecting groups are removed;
E. Conversion of the uracil moiety 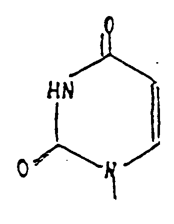 in a compound corresponding to that illustrated in the formula of claim 1 to the cytosine moiety 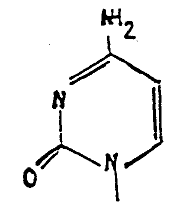 whereafter the cytosine derivative thus obtained, if desired, is converted to a therapeutically acceptable salt.

## Patentansprüche

1. Verbindung der Formel 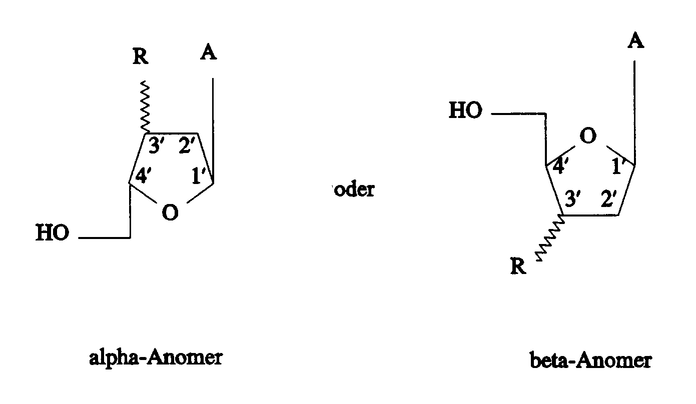 wobei die Reste A und R wie folgt definiert sind: 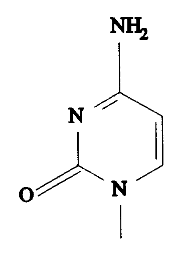
R: -CH₂OH,
und wobei R relativ zu der Hydroxymethyl-Funktion an der 4'-Position entweder cis-Konfiguration oder trans-Konfiguration aufweist, und ihre therapeutisch verträglichen Salze.

2. Die Verbindung nach Anspruch 1, wobei R an der 3'-Position und der Hydroxymethyl-Rest und der 4'-Position die trans-Konfiguration haben.

3. Die Verbindung nach Anspruch 1 oder 2 und ihre therapeutisch verträglichen Salze für die Anwendung in der Therapie.

4. Pharmazeutische Zusammensetzung, umfassend als aktiven Bestandteil eine Verbindung nach Anspruch 1 oder 2 oder ihre therapeutisch verträglichen Salze.

5. Verwendung der Verbindung nach Anspruch 1 oder 2 und ihrer therapeutisch verträglichen Salze für die Herstellung eines Medikaments für die therapeutische und/oder prophylaktische Behandlung von Infektionen, die von einem Retrovirus wie HIV oder von dem Hepatitis B-Virus (HBV) verursacht werden.

6. Die Verwendung nach Anspruch 5, wobei die therapeutische Behandlung gegen von HIV verursachte Infektionen im Menschen gerichtet ist.

7. Die Verwendung nach Anspruch 5, wobei die therapeutische Behandlung gegen von HBV verursachte Infektionen im Menschen gerichtet ist.

8. Verfahren zur Herstellung der Verbindung nach Anspruch 1 oder 2 oder von deren therapeutisch verträglichem Salz, wobei das Verfahren ausgewählt ist aus:
A. ein Glykosid, wie es in der Formel von Anspruch 1 enthalten ist, an die N-1-Position des Cytosins zu kondensieren, woraufhin das α-Anomer der derart gebildeten Verbindung abgetrennt und jegliche Schutzgruppen entfernt werden;
B. Anomerisierung eines β-Anomeren der Formel 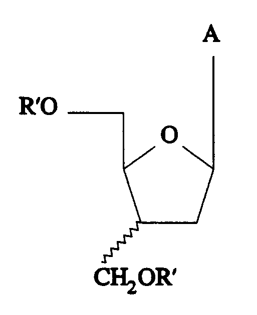 wobei R' eine die Hydroxy-Gruppe schützende Gruppe ist, zu einem Gemisch aus α- und β-Anomeren, woraufhin das α-Anomer abgetrennt und jegliche Schutzgruppen entfernt werden;
C. Transglykosylierung eines Nukleosids der Formel 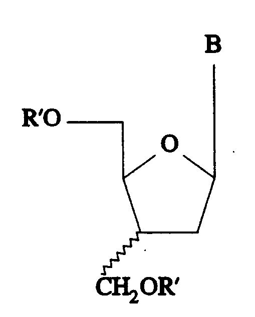 wobei R' wie oben definiert und B eine Pyrimiden- oder Purinbase ist, unter Bildung eines den Cytosin-Rest als A enthaltenden Nukleosids, woraufhin das α-Anomer abgetrennt und jegliche Schutzgruppen entfernt werden;
D. Substitution des Rests R'' in einer Verbindung der Formel 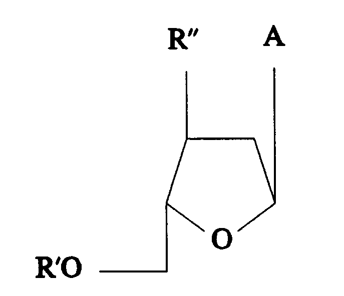 wobei A und R' wie oben definiert und R'' eine Anstrittsgruppe sind, durch den Rest -CH₂OR' als R, woraufhin jegliche Schutzgruppen entfernt werden;
E. Umwandlung der Uracil-Einheit 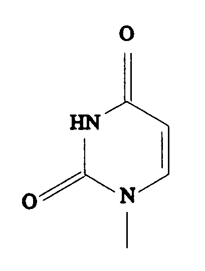 in einer Verbindung, die der in der Formel von Anspruch 1 dargestellten entspricht, in die Cytosin-Einheit 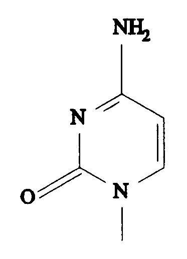 woraufhin das so erhaltene Cytosin-Derivat, sofern dies erwünscht ist, in ein therapeutisch verträgliches Satz umgewandelt wird.

## Revendications

1. Un composé de formule 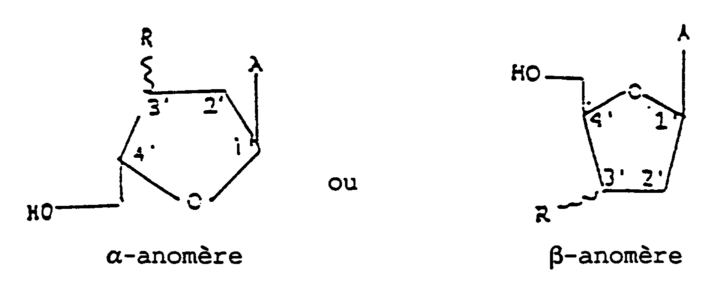 dans laquelle les radicaux A et R sont comme suit :
A : 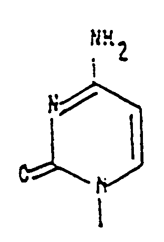
R: =-CH₂OH,
et dans laquelle R peut avoir soit une configuration cis soit une configuration trans par rapport à la fonction hydroxy-méthyle en position 4' et ses sels acceptables du point de vue thérapeutique.

2. Un composé selon la revendication 1, dans lequel R en position 3' et hydroxy-méthyle en position 4' ont la configuration trans.

3. Un composé selon la revendication 1 ou 2, et les sels acceptables du point de vue thérapeutique de celui-ci à usage thérapeutique.

4. Une composition pharmaceutique comprenant comme ingrédient actif un composé selon la revendication 1 ou 2 ou ses sels acceptables du point de vue thérapeutique.

5. Utilisation d'un composé selon la revendication 1 ou 2, et de ses sels acceptables du point de vue thérapeutique dans la fabrication d'un médicament pour le traitement thérapeutique et/ou prophylactique d'infections provoquées par un rétrovirus comme HIV ou par le virus de l'hépatite B (HBV).

6. L'utilisation selon la revendication 5, dans laquelle ledit traitement thérapeutique est contre l'infection chez l'homme provoquée par HIV.

7. L'utilisation selon la revendication 5, dans laquelle ledit traitement thérapeutique est contre l'infection chez l'homme provoquée par HBV.

8. Un procédé pour la préparation d'un composé selon la revendication 1 ou 2 ou de ses sels acceptables du point de vue thérapeutique choisi à partir du groupe comprenant:
A. Condensation d'un glycoside répondant à la formule de la revendication 1 en position N-1 de la cytosine, après quoi l' α-anomère du composé ainsi formé est séparé et tous les groupes protecteurs sont éliminés;
B. Anomérisation d'un β-anomère de formule 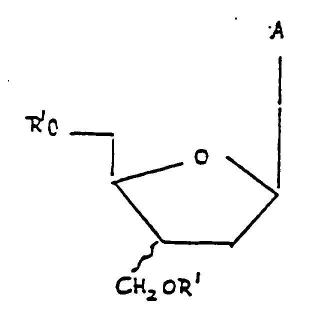 dans laquelle R' est un groupe protecteur hydroxy d'un mélange d' α et β-anomères, après quoi l' α-anomère est séparé et tous les groupes protecteurs sont éliminés ;
C. Transglycosylation d'un nucléoside de formule 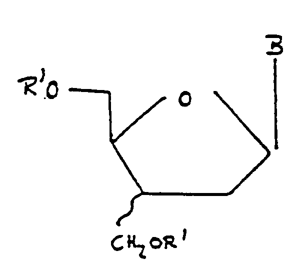 dans laquelle R' est défini comme ci-dessus et dans laquelle B est une pyrimidine ou une base purine, avec formation d'un nucléoside contenant le radical cytosine comme A, après quoi l' α-anomère est séparé et tous les groupes protecteurs sont éliminés ;
D. Substitution du radical R'' dans un composé de formule 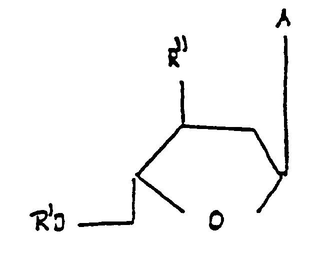 dans laquelle A et R' sont comme définis ci-dessus et dans laquelle R'' est un groupe qui part avec le radical - CH₂OR' comme R, après quoi tous les groupes protecteurs sont éliminés ;
E. Conversion de la fraction uracile : 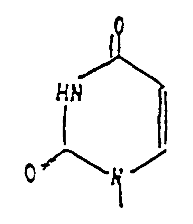 selon un composé correspondant à celui illustré dans la formule de la revendication 1 en la fraction cytosine : 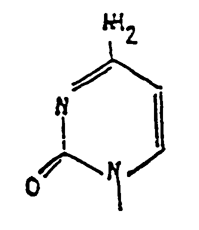 après quoi le dérivé de cytosine ainsi obtenu, si désiré, est transformé en un sel acceptable du point de vue thérapeutique.
